# EUROPÄISCHE PATENTSCHRIFT

(11) **EP 2 934 406 B1**
(45) Veröffentlichungstag und Bekanntmachung des Hinweises auf die Patenterteilung: **15.02.2017**
(21) Anmeldenummer: 13805405.1
(22) Anmeldetag: 16.12.2013
(51) Int. Cl.: A61F 13/505, A61F 13/49, A61F 13/539

(54) **ABSORBIERENDER INKONTINENZARTIKEL MIT WINDELHÜLLE UND ABSORPTIONSEINLAGE**
ABSORBENT INCONTINENCE ARTICLE HAVING A DIAPER COVER AND ABSORPTION INSERT
ARTICLE ABSORBANT POUR INCONTINENCE MUNI D'UNE ENVELOPPE DE COUCHE ET D'UNE GARNITURE ABSORBANTE

(30) Priorität: 21.12.2012 DE 102012025437
(43) Veröffentlichungstag der Anmeldung: 28.10.2015
(73) Patentinhaber: Paul Hartmann AG, 89522 Heidenheim (DE)
(72) Erfinder: KNECHT, Theresia, 73433 Aalen (DE)
(86) Internationale Anmeldenummer: PCT/EP2013/076710
(87) Internationale Veröffentlichungsnummer: WO 2014/095727

(56) Entgegenhaltungen:
- WO-A1-99/33427
- WO-A1-2007/000315
- WO-A1-2010/108660
- WO-A1-2012/054591
- JP-A- 2001 087 312
- US-A- 5 593 400

## Beschreibung

Die vorliegende Erfindung betrifft eine absorbierende Inkontinenzwegwerfwindel des offenen Typs mit einem Rückbereich, einem Vorderbereich und einem dazwischen liegenden Schrittbereich und mit einem Hauptteil mit einer in Gebrauch dem Körper zugewandten Innenseite und einer in Gebrauch dem Körper abgewandten Außenseite, und mit an den ersten und zweiten Seitenrand des Hauptteils angefügten diskreten Seitenteilen. Die Inkontinenzwegwerfwindel ist für Erwachsene vorgesehen und ist als Wegwerfwindel ausgebildet, das heißt zum einmaligen Gebrauch bestimmt.

Derartige Inkontinenzwegwerfwindeln sind beispielsweise auch aus der WO2004/105668A1 bekannt.

Bei derartigen Inkontinenzwegwerfwindeln können die erwähnten Seitenteile aus einem anderen Material gebildet sein als der Hauptteil. Beispielsweise können die Seitenteile, die häufig auch als "Ohren" der Inkontinenzwegwerfwindel bezeichnet werden, atmungsaktiv, insbesondere luft- und wasserdampfdurchlässig ausgebildet werden, wohingegen der Hauptteil flüssigkeitsundurchlässig ausgeführt ist.

Zum Schließen der Inkontinenzwegwerfwindel werden die vorzugsweise unlösbar am Rückbereich angefügten Seitenteile auf die Bauchseite des Benutzers geschlagen und dort entweder mit der Außenseite des Vorderbereichs des Hauptteils oder mit der Außenseite der Seitenteile des Vorderbereichs lösbar verbunden.

Insoweit eine derartige Inkontinenzwegwerfwindel mit mechanischen Verschlusshilfen ausgestattet wird, resultiert das Problem, dass für die üblicherweise an den hinteren Seitenteilen angeordneten meist als Kletthaken ausgeführten Verschlusshilfen ein entsprechender Landebereich an der Außenseite des Vorderbereiches der Windel vorgesehen sein muss, der mit den Kletthaken in Eingriff bringbar sein muss.

Üblicherweise wird die Außenseite des Hauptteils derartiger Inkontinenzwegwerfwindeln allerdings durch ein Folienmaterial gebildet, um ein Austreten von Flüssigkeit durch den Saugkörper hindurch nach außen zu verhindern. Die Seitenteile derartiger Inkontinenzwegwerfwindeln sind vorzugsweise aus glatten Vliesmaterialien gebildet, um dort, wo keine sichere Flüssigkeitsbarriere erforderlich ist, die Hautfreundlichkeit der Windel zu verbessern. Somit würde eine Landefläche zum sicheren Festlegen der Kletthaken auf der Außenseite des Vorderbereichs der Windel das Anbringen eines zusätzlichen Materials, insbesondere eine an sich bekannte textile Loop-Komponente erfordern. Derartige Loop-Komponenten müssten sich allerdings über weite Teile des Vorderbereiches der Außenseite der Windel erstrecken, um das bei Inkontinenzwegwerfwindeln (Windeln für Erwachsene) erforderliche hohe Maß an Flexibilität in der Größenanpassung zu gewährleisten. Da textile Loop-Komponenten einen wesentlichen Kostenfaktor bilden, verbietet sich eine derartige Lösung schon aus wirtschaftlichen Gründen.

Des Weiteren hat sich bei Inkontinenzwegwerfwindeln der bekannten Art gezeigt, dass das subjektive Empfinden des Komforts der Windel trotz der hautfreundlichen und luft- wie wasserdampfdurchlässigen Seitenteile deutliche Unterschiede ausweist.

Bereits mit der DE102009016381A1 wurde vorgeschlagen, den Hauptteil als eine integrale Einheit zu bilden aus einem Topsheet, einem Vlies-Folienlaminat als Backsheet und einem dazwischen angeordneten Absorptionskörper. An diesen Hauptteil sind hinten und vorn vier diskrete Seitenteile angefügt.

Es ist Aufgabe der vorliegenden Erfindung eine alternative Inkontinenzwegwerfwindel bereitzustellen, welche sich wirtschaftlicher herstellen lässt und darüber hinaus dem Anwender der Inkontinenzwegwerfwindel ein höheres Maß an Tragkomfort gewährleistet.

Es wird mithin vorgeschlagen eine absorbierende Inkontinenzwegwerfwindel des offenen Typs mit einer zumindest dreiteiligen Windelhülle mit einer Innenseite und einer Außenseite, wobei die Windelhülle einen Hauptteil und zumindest zwei separate hintere Seitenteile umfasst, wobei der Hauptteil einen Vorderbereich, einen Rückbereich und einen in Längsrichtung dazwischen liegenden und zwischen den Beinen eines Benutzers zu liegen kommenden Schrittbereich aufweist, und wobei die hinteren Seitenteile an dem Rückbereich des Hauptteils beidseitig derart angeordnet sind, dass die hinteren Seitenteile in ersten Überlappungsbereichen mit ihrer Außenseite auf der Innenseite des Hauptteils entlang hinterer seitlicher Längsränder festgelegt sind, und wobei sich die hinteren Seitenteile in Querrichtung über die hinteren seitlichen Längsränder des Hauptteils hinaus erstrecken, und wobei die hinteren Seitenteile Verschlussmittel aufweisen, und wobei der Hauptteil und die hinteren Seitenteile ein Vliesstoffmaterial umfassen oder daraus gebildet sind, und wobei die Windelhülle sich kontinuierlich über eine Länge L1 von einem vorderen Ende der Windelhülle über den Schrittbereich bis zu einem hinteren Ende der Windelhülle erstreckt, und wobei auf der Innenseite der Windelhülle eine Absorptionseinlage mit einer Innenseite und einer Außenseite angeordnet ist, wobei die Absorptionseinlage ein flüssigkeitsundurchlässiges Backsheet und ein Topsheet umfasst, zwischen denen ein Absorptionskörper angeordnet ist, wobei die Absorptionseinlage sich in die ersten Überlappungsbereiche hinein erstreckt und dort mit ihrer Außenseite auf der Windelhülle zumindest abschnittsweise festgelegt ist, wobei die Absorptionseinlage eine Länge L2 aufweist und wobei gilt L2 < L1. Die Längserstreckung der Absorptionseinlage ist also geringer als die der Windelhülle.

Die Windelhülle bildet somit als ein zumindest dreiteiliger (2 Seitenteile und der Hauptteil) Vliesstoffverbund ein überaus angenehm zu tragendes Windelchassis.

In den ersten Überlappungsbereichen überfangen die inneren Längsränder der Seitenteile die hinteren Längsränder des Hauptteils und sind dort mit ihrer Außenseite auf der Innenseite des Hauptteils vorzugsweise unlösbar festgelegt. Mit der Innenseite eines vor- oder nachstehend beschriebenen Flachmaterials ist jeweils die in Gebrauch der Windel körperzugewandte Seite des Flachmaterials gemeint. Entsprechend ist mit der Außenseite eines vor- oder nachstehend beschriebenen Flachmaterials jeweils die in Gebrauch der Windel körperabgewandte Seite gemeint.

Das den Hauptteil der Windelhülle bildende Flachmaterial besteht im Wesentlichen gänzlich aus Vliesstoffmaterial. Der Hauptteil kann daher über seine gesamte Erstreckung luftdurchlässig gestaltet werden. Die als integrale Einheit ausgebildete Absorptionseinlage hingegen umfasst ein Folienmaterial als Backsheet. Dadurch ist sichergestellt, dass in Gebrauch keine Flüssigkeit aus dem Absorptionskörper nach außen gelangen kann. Da die Längserstreckung der Absorptionseinlage L2 geringer ist als die Längserstreckung L1 der Windelhülle, kann die erfindungsgemäße Windel im Vergleich zur in DE102009016381A1 offenbarten Inkontinenzwegwerfwindel mit deutlich geringerem Materialeinsatz gefertigt werden. Zudem bleibt die Luftdurchlässigkeit der Windelhülle außerhalb der flächenhaften Erstreckung der Absorptionseinlage in Gebrauch voll erhalten. Die Erfinder haben außerdem erkannt, dass der Verbund der Windelhülle weiter verfestigt werden kann, wenn die ersten Überlappungsbereiche der Windelhülle, also die Bereiche, in denen der Hauptteil und die hinteren Seitenteile in überlappender Konfiguration unlösbar verbunden sind, durch die Absorptionseinlage zumindest teilweise überfangen werden, sich die Absorptionseinlage also in die ersten Überlappungsbereiche hinein erstreckt und dort mit ihrer Außenseite auf der Innenseite der Windelhülle, also im Überlappungsbereich auf einer Innenseite der Seitenteile festgelegt ist. Hierdurch ist die Gefahr des ungewollten Ausreißens der Seitenteile aus dem aus Seitenteilen und Hauptteil bestehenden Vliesstoffverbund deutlich reduziert.

Nach einer Weiterbildung der Erfindung ist vorgesehen, in gleicher Weise wie an den Rückbereich auch an den Vorderbereich des Hauptteils links und rechts (beidseitig) Seitenteile anzufügen, also derart, dass die vorderen Seitenteile in zweiten Überlappungsbereichen mit den Seitenrandbereichen des Hauptteils verbunden sind. Vorzugsweise erstreckt sich die Absorptionseinlage solchenfalls auch in die zweiten Überlappungsbereiche hinein und ist dort mit ihrer Außenseite an einer Innenseite der Windelhülle festgelegt.

Vorzugsweise bilden das Backsheet und/oder das Topsheet in Querrichtung und/oder in Längsrichtung einen Überhang über den Absorptionskörper. Es sind solchenfalls insbesondere Teilbereiche des Überhangs der Absorptionseinlage, welche sich in die ersten und/oder zweiten Überlappungsbereiche hinein erstrecken.

Es hat sich hinsichtlich der Verstärkung des Vliesstoffverbundes, welcher den Hauptteil bildet, als vorteilhaft erwiesen, wenn jeweils mindestens 20%, insbesondere mindestens 30%, weiter insbesondere höchstens 90 %, weiter insbesondere höchstens 80% der Fläche der ersten Überlappungsbereiche mit der Absorptionseinlage verbunden ist.

Zur Ermittlung von Abmessungen von Komponenten der Inkontinenzwegwerfwindel und der Flächenanteile wird die Inkontinenzwegwerfwindel in plan ausgebreitetem Zustand vermessen.

Das Verhältnis der Längen L2/L1 ist vorzugsweise kleiner als 0,9, insbesondere kleiner als 0,8, weiter insbesondere kleiner als 0,7, weiter insbesondere größer als 0,3.

Der Anteil der mit der Absorptionseinlage verbundenen Fläche der zweiten Überlappungsbereiche ist vorzugsweise geringer ist als der Anteil der mit der Absorptionseinlage verbunden Fläche der ersten Überlappungsbereiche. Wegen der Anordnung der Verschlussmittel an den hinteren Seitenteilen und der damit einhergehenden Gefahr durch über die Verschlussmittel in die Überlappungsbereiche eingeleiteten Zugkräfte den Verbund zwischen Seitenteilen und Hauptteil zu zerstören, hat es sich als vorteilhaft erwiesen, die ersten Überlappungsbereiche stärker zu verfestigen als die zweiten Überlappungsbereiche.

Vorteilhaft ist, wenn jeweils mindestens 10%, insbesondere mindestens 20%, weiter insbesondere höchstens 85 %, weiter insbesondere höchstens 75% der Fläche der zweiten Überlappungsbereiche mit der Absorptionseinlage verbunden ist.

Zur Verfestigung des den Hauptteil bildenden Vliesstoffverbundes hat es sich als besonders vorteilhaft erwiesen, wenn die Absorptionseinlage die ersten und/oder die zweiten Überlappungsbereiche in Querrichtung vollständig überfängt. Vorzugsweise ist die Absorptionseinlage hierzu in Querrichtung zumindest in dem Rückbereich der Windelhülle, insbesondere aber über ihre gesamte Längserstreckung durchgehend breiter ausgebildet als der Hauptteil der Windelhülle. Vorzugsweise erstreckt sich die Absorptionseinlage in Querrichtung um das Maß D nach außen über die hinteren, insbesondere über die hinteren und vorderen seitlichen Längsränder des Hauptteils hinweg, wobei D vorzugsweise 0,5-10,0 cm, insbesondere 1,0- 8,0 cm, weiter insbesondere 1,2-5,0 cm beträgt.

Die Absorptionseinlage überlappt somit vorzugsweise entlang zumindest einer gedachten, in Querrichtung verlaufenden Linie sowohl einen jeweiligen inneren Längsrand der hinteren Seitenteile als auch einen jeweiligen hinteren seitlichen Längsrand des Hauptteils und ist entlang zumindest dieser Linie mit der Innenseite der Windelhülle verbunden, das heißt darauf festgelegt.

Die Außenseite der Absorptionseinlage kann vorzugsweise in weiteren Bereichen, also auch außerhalb der ersten oder zweiten Überlappungsbereiche vorzugsweise auch vollflächig mit der Innenseite der Windelhülle verbunden sein, insbesondere durch kontinuierlichen oder diskontinuierlichen Holtmeltklebstoffauftrag, zum Beispiel in Form eines Spiralsprühklebemusters.

Weiter vorzugsweise sind die flächenhafte Erstreckung des Absorptionskörpers und deren Anordnung derart ausgeführt, dass sich der Absorptionskörpers nicht in die ersten und/oder zweiten Überlappungsbereiche hinein erstreckt.

Die Absorptionseinlage kann vorteilhaft eine zumindest abschnittsweise gerundete, also kurvenförmige Beinöffnungskontur aufweisen, derart dass die Absorptionseinlage in einem Bereich auf Höhe der Quermittelachse der Windelhülle eine geringere Quererstreckung aufweist als in dem Rücken- und/oder Bauchbereich.

Die hinteren Seitenteile und/oder die vorderen Seitenteile und/oder der Hauptteil der Windelhülle können jeweils aus rechteckförmigen Abschnitten gebildet sein.

Weiter vorteilhaft kann die Windelhülle aber eine zumindest abschnittsweise gerundete, also kurvenförmige Beinöffnungskontur aufweisen, derart dass die Windelhülle in einem Bereich auf Höhe der Quermittelachse der Windelhülle eine geringere Quererstreckung aufweist als in dem Rücken- und/oder Bauchbereich. Hierbei können der Hauptteil und/oder die vorderen Seitenteile und/oder die hinteren Seitenteile eine kurvenförmige Beinöffnungskontur aufweisen.

In den ersten und/oder zweiten Überlappungsbereichen sind die Seitenteile mit ihrer Außenseite in überlappender Konfiguration mit der Innenseite der Windelhülle verbunden, also aneinandergefügt. Das Aneinanderfügen kann vorteilhaft durch an sich bekannte Fügemittel wie Klebstoffe, insbesondere Hotmeltklebstoffe, durch Verprägen, durch thermisches Kalandrieren, oder durch Schweißen, insbesondere durch Ultraschallschweißen erfolgen.

Das Aneinanderfügen der Außenseite der Absorptionseinlage mit der Innenseite der Windelhülle in den ersten und/oder zweiten Überlappungsbereichen erfolgt ebenfalls vorteilhaft durch Klebstoffe, insbesondere Hotmeltklebstoffe, durch Verprägen, durch thermisches Kalandrieren, oder durch Schweißen, insbesondere durch Ultraschallschweißen.

An den hinteren Seitenteilen sind Verschlussmittel vorgesehen. Die Verschlussmittel weisen beispielsweise klebende, insbesondere aber mechanische Verschlusselemente wie Kletthaken auf. Besonders bevorzugt weisen die Verschlussmittel sowohl klebende als auch mechanische Verschlusselemente auf. Vorteilhaft sind hierzu auf einem jeweiligen Verschlussstreifen nebeneinander angeordnete klebende Bereiche und mechanisch haftende Bereiche vorgesehen, so wie dies beispielsweise in EP321232A1 beschrieben ist.

Zum bestimmungsgemäßen Festlegen der Inkontinenzwegwerfwindel an den Körper eines Menschen sind die Verschlussmittel auf der Außenseite eines vorderen Bereiches der Windel festlegbar, wobei der Anwender die Möglichkeit besitzt, die Windel an die anatomischen Gegebenheiten des Nutzers, insbesondere an den Hüftumfang des Nutzers der Windel anzupassen. Hierzu ist - im Gegensatz zu den bekannten Fix-Point-Fasteners - vorgesehen, dass der Anwender die Möglichkeit besitzt, die Positionierung der Verschlussmittel auf der Außenseite eines vorderen Bereiches der Windel hinsichtlich der Querrichtung zu variieren. Vorzugsweise sind die Verschlussmittel auf der gesamten Außenseite des Vorderbereichs des Hauptteils festlegbar. Weiter vorzugsweise sind die Verschlussmittel auf der gesamten Außenseite der vorderen Seitenteile festlegbar, insoweit die Inkontinenzwegwerfwindel vordere Seitenteile aufweist.

Nach einer bevorzugten Ausführungsform ist vorgesehen, dass die Verschlussmittel der hinteren Seitenteile zum bestimmungsgemäßen Festlegen der Inkontinenzwegwerfwindel an den Körper eines Menschen zumindest bereichsweise sowohl an der Außenseite des Hauptteils im Vorderbereich als auch an der Außenseite der vorderen Seitenteile lösbar festlegbar sind. Vorzugsweise sind hierbei die Haltekräfte zwischen den Verschlussmitteln und der Außenseite der vorderen Seitenteile größer als die Haltekräfte zwischen den Verschlussmitteln und der Außenseite des Hauptteils. Dies veranlasst den Benutzer in der Mehrzahl der Fälle, die Verschlussmittel an den vorderen Seitenteilen festzulegen, was der Passform der Windel zugute kommt. Die Haltekräfte als Über-Bauch-Haltekräfte ermittelt zwischen den insbesondere mechanischen Verschlusshilfen aufweisenden Verschlussmitteln und der Außenseite des Hauptteils betragen vorzugsweise 20-57 N/25mm, insbesondere 25-50 N/25mm, gemessen nach der in EP1915977A1 beschriebenen Prüfmethode.

Es hat sich weiter als vorteilhaft erwiesen, die vorderen und/oder hinteren Seitenteile und/oder den Hauptteil aus einem Vliesstoffmaterial zu bilden, welches zumindest eine Rezepturkomponente auf Basis eines thermoplastischen Polymers enthält. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können.

Vorteilhaft unterscheiden sich die hinteren Seitenteile von den vorderen Seitenteilen und/oder dem Hauptteil bezüglich mindestens einer, insbesondere mindestens zweier, weiter insbesondere mindestens dreier und weiter insbesondere mindestens vierer ihrer Primäreigenschaften ausgewählt aus der Gruppe Art des Materials, Flächengewicht, Atmungsaktivität, Dichte, Dehnbarkeit, Verschlusskraft, Flächenerstreckung, Dicke, Farbe.

Art des Materials: Es erweist sich als vorteilhaft, wenn die vorderen Seitenteile aus einem weicheren, hautfreundlicheren Vliesmaterial gebildet sind als die hinteren Seitenteile, da die vorderen Seitenteile beim Anlegen der Windel an den Körper bestimmungsgemäß innen zu liegen kommen. Weiterhin kann es vorteilhaft sein, die hinteren Seitenteile aus einem zugfesteren Material zu bilden, da die Verschlussmittel an den hinteren Seitenteilen angebracht sind und beim Anlegen der Windel starke Zugkräfte über die Verschlussmittel auf die Seitenteile einwirken. Bevorzugte Differenzierungen hinsichtlich der Art des Materials lassen sich durch die verwendete Faserart, das Vliesbildungsverfahren oder Laminatbildungen realisieren.

Flächengewicht: Die zuvor genannten Anforderungen können vorzugsweise zumindest anteilig über eine Differenzierung des Flächengewichtes, gemessen in g/m2, erreicht werden. Vorzugsweise unterscheidet sich das Flächengewicht der vorderen Seitenteile um mindestens 10%, insbesondere um mindestens 20% und weiter insbesondere um mindestens 30% von dem der hinteren Seitenteile. Weiter vorzugsweise unterscheidet sich das Flächengewicht der vorderen und/oder hinteren Seitenteile um mindestens 10%, insbesondere um mindestens 20% und weiter insbesondere um mindestens 30% von dem Flächengewicht des Hauptteils der Windelhülle.

Das Flächengewicht der vorderen und/oder der hinteren Seitenteile und/oder des Hauptteils der Windelhülle beträgt vorzugsweise 10-60 g/m², insbesondere 12-45 g/m², weiter insbesondere 15-40 g/m² und weiter insbesondere 18-35 g/m².

Atmungsaktivität: Da das subjektive Empfinden der Beeinträchtigung des Tragekomforts von Zielgruppe zu Zielgruppe (beispielsweise bettlägerige Patienten vs. mobile Patienten) verschieden ist, kann es vorteilhaft sein, die Atmungsaktivität entweder der vorderen oder der hinteren Seitenteile höher auszubilden. Vorzugsweise unterscheidet sich die Atmungsaktivität gemessen als Wasserdampfdurchlässigkeit (WVTR nach DIN 53 122-1 Ausgabe: 2001-08) der vorderen Seitenteile von der der hinteren Seitenteile um mindestens 5%, insbesondere um mindestens 10% und weiter insbesondere um mindestens 20%. Vorzugsweise beträgt dabei die Atmungsaktivität der vorderen und/oder hinteren Seitenteile mindestens 1000 g/m²/24h, insbesondere mindestens 1500 g/m²/24h, weiter vorzugsweise mindestens 2000 g/m²/24h.

Dichte und Dicke: Die subjektiv empfundene Weichheit des Seitenteilmaterials und damit eine wesentliche Komponente des Tragekomforts kann vorteilhaft auch über eine Differenzierung der Dichte und/oder Dicke des Materials gesteuert werden. Vorzugsweise unterscheidet sich die Dicke gemessen in mm, ermittelt bei einem Prüfdruck von 0,5 kPa, und/oder die Dichte gemessen in g/cm³, ermittelt aus den Größen Flächengewicht und Dicke des Materials, der vorderen Seitenteile um mindestens 15%, insbesondere um mindestens 20% und weiter insbesondere um mindestens 25% von der Dichte und/oder der Dicke der hinteren Seitenteile. Weiter vorzugsweise unterscheidet sich die Dicke und/oder die Dichte der vorderen und/oder der hinteren Seitenteile um mindestens 15%, insbesondere um mindestens 20% und weiter insbesondere um mindestens 25% von der Dichte und/oder der Dicke des Hauptteils der Windelhülle.

Dehnbarkeit: Unter Dehnung wird vorliegend das Verhältnis zwischen einer Längenzunahme eines Seitenteils der Inkontinenzwegwerfwindel infolge einer Krafteinwirkung und der ursprünglichen Länge verstanden. In Gebrauch derartiger Inkontinenzwegwerfwindeln wirken auf die Seitenteile insbesondere Kräfte in Umfangs- also Windelquerrichtung. Mit der Eigenschaft Dehnbarkeit ist somit das Ausmaß der Dehnung des Seitenteils bei Einwirken einer Kraft in Windelquerrichtung gemeint. Das heißt, je höher das Ausmaß der Dehnung, desto höher ist die Dehnbarkeit. Vorzugsweise weist ein hinteres Seitenteil bei in Gebrauch der Windel üblicher Krafteinwirkung eine größere Dehnbarkeit auf als ein vorderes Seitenteil. Insbesondere weist nach der in DE102005048868A1 beschriebenen Prüfmethode ein hinteres Seitenteil bei einer Krafteinwirkung von 45 N eine höhere Dehnung auf als ein vorderes Seitenteil. Vorzugsweise weist ein hinteres Seitenteil bei einer Krafteinwirkung von 45 N eine Dehnung von mindestens 20%, insbesondere mindestens 25% und weiter insbesondere mindestens 30% auf. Ein vorderes Seitenteil hingegen weist bei einer Krafteinwirkung von 45 N lediglich eine Dehnung von vorzugsweise höchstens 15%, insbesondere höchstens von 10% und weiter insbesondere von höchstens 8% auf. Vorzugsweise ist zumindest ein hinteres Seitenteil zumindest in Querrichtung elastisch dehnbar. Die Dehnbarkeit des Seitenteils wird als elastisch bezeichnet, wenn bei kurzzeitiger Einwirkung einer Kraft (2-5 Sekunden) eine Dehnung von mindestens 40% möglich ist und bei Wegnahme dieser Kraft eine Dehnung (bleibende Dehnung) von höchstens 20% verbleibt. In einer vorteilhaften Weiterbildung der Erfindung beträgt die elastische Dehnbarkeit eines hinteren Seitenteils in Querrichtung mindestens 40%, insbesondere mindestens 50%. Nach einem weiteren
Erfindungsgedanken beträgt das absolute Ausmaß der elastischen Dehnung eines hinteren Seitenteils mindestens 3 cm, insbesondere mindestens 5 cm und weiter insbesondere mindestens 7 cm.

Verschlusskraft: Unter Verschlusskraft der Seitenteile wird die Haltekraft zwischen den Verschlussmitteln der hinteren Seitenteile und der Außenseite der Seitenteile verstanden. Vorzugsweise sind die Haltekräfte zwischen den Verschlussmitteln und der Außenseite der hinteren Seitenteile hierbei geringer als die Haltekräfte zwischen den Verschlussmitteln und der Außenseite der vorderen Seitenteile. Dies führt in vorteilhafter Weise dazu, dass der Benutzer die Verschlussmittel bevorzugt an den vorderen Seitenteilen festlegt, was Passform und Tragekomfort der Windel deutlich zugute kommt. Die vorstehend oder nachfolgend erwähnten Haltekräfte werden vorzugsweise als Über-Bauch-Haltekräfte ermittelt. Die Über-Bauch-Haltekräfte sind im Rahmen dieser Erfindung nach der in EP 1915977A1 beschriebenen Prüfmethode zu bestimmen. Die Haltekräfte als Über-Bauch-Haltekräfte ermittelt zwischen den insbesondere mechanischen Verschlusshilfen aufweisenden Verschlussmitteln und der Außenseite der vorderen Seitenteile betragen vorzugsweise 58-90N/25mm, insbesondere 60-80N/25mm.

Die Über-Bauch-Haltekräfte zwischen den insbesondere mechanischen Verschlusshilfen aufweisenden Verschlussmitteln und der Außenseite der hinteren Seitenteile sind vorzugsweise geringer als die Über-Bauch-Haltekräfte zwischen den Verschlussmitteln und der Außenseite der vorderen Seitenteile, sie betragen vorzugsweise aber gleichwohl mindestens 15 N/25mm, insbesondere mindestens 30 N/25mm.

Flächenerstreckung: In Weiterbildung der Erfindung erweist es sich als vorteilhaft, wenn die hinteren Seitenteile eine größere, vorzugsweise eine um mindestens 10 %, insbesondere eine um mindestens 15% größere Flächenerstreckung aufweisen als die vorderen Seitenteile. Insbesondere kann die Länge der hinteren Seitenteile, also deren Erstreckung in Windellängsrichtung mindestens 10 cm, insbesondere mindestens 15 cm, weiter insbesondere mindestens 18 cm und weiter insbesondere mindestens 22 cm betragen. Es erweist sich weiterhin als vorteilhaft, wenn die Länge der hinteren Seitenteile mindestens 10 %, insbesondere mindestens 15%, weiter insbesondere mindestens 20% und weiter insbesondere mindestens 22% der Gesamtlänge L1 der Windelhülle beträgt. Vorteilhafterweise beträgt die Gesamtlänge L1 der Windelhülle 50-120 cm, insbesondere 60-110 cm und weiter insbesondere 70-110 cm.

Weiterhin erweist es sich als vorteilhaft, wenn die vorderen Seitenteile eine geringere, insbesondere eine um mindestens 5%, weiter insbesondere eine um mindestens 10%, weiter insbesondere eine um mindestens 15% und weiter insbesondere eine um höchstens 50% geringere Längserstreckung aufweisen als die hinteren Seitenteile.

Weiterhin erweist es sich als vorteilhaft, wenn die Breite der Seitenteile, also die Erstreckung der Seitenteile in Querrichtung über den hinteren seitlichen Längsrand des Hauptteils hinaus 10-40 cm, insbesondere 12-30 cm, weiter insbesondere 13-25 cm beträgt. Vorzugsweise weisen die vorderen Seitenteile die gleiche Breite auf wie die hinteren Seitenteile.

Farbe: Schließlich kann es vorteilhaft sein, die vorderen von den hinteren Seitenteilen hinsichtlich der Farbe zu differenzieren. Auch dies kann den Benutzern die Funktion der vorderen Seitenteile als bevorzugte Landefläche der Verschlussmittel verdeutlichen.

Der modulare Aufbau der erfindungsgemäßen Inkontinenzwegwerfwindeln erlaubt es dem Windelhersteller, unterschiedlichen Anforderungen der Anwender insbesondere hinsichtlich Passform und Absorptionsvermögen nachzukommen, ohne die Produktionstechnik zur Fertigung der Inkontinenzwegwerfwindeln über Gebühr individualisieren zu müssen.

Nach einem weiteren Erfindungsgedanken ist daher vorgesehen, eine Gruppe von Inkontinenzwegwerfwindeln bereitzustellen, welche zumindest erste und zweite wie vorstehend beschriebene erfindungsgemäße Inkontinenzwegwerfwindeln umfasst, wobei die ersten und zweiten Inkontinenzwegwerfwindeln hinsichtlich der Abmessungen in Längs- und/oder Querrichtung, insbesondere auch hinsichtlich weiterer Eigenschaften wie Materialzusammensetzung oder Flächengewicht des Hauptteils und/oder der hinteren Seitenteile, insbesondere hinsichtlich aller Eigenschaften eine identische Windelhülle aufweisen, wobei die Absorptionseinlage der ersten Inkontinenzwegwerfwindeln sich von der Absorptionseinlage der zweiten Inkontinenzwegwerfwindeln zumindest in einer Eigenschaft, ausgewählt aus der Gruppe, Abmessungen in Längs- und/oder Querrichtung, Absorptionsvermögen (gemessen nach ISO 11948-1 (1996)) Materialzusammensetzung, Schichtaufbau, Elastifizierung unterscheidet.

Nach einem weiteren Erfindungsgedanken ist vorgesehen, eine Gruppe von Inkontinenzwegwerfwindeln bereitzustellen, welche erste und zweite erfindungsgemäße Inkontinenzwegwerfwindeln umfasst, wobei die ersten und zweiten Inkontinenzwegwerfwindeln hinsichtlich der Abmessungen in Längs- und/oder Querrichtung, und vorzugsweise auch hinsichtlich weiterer Eigenschaften wie Materialzusammensetzung oder Flächengewicht, weiter insbesondere hinsichtlich aller Eigenschaften eine identische Absorptionseinlage aufweisen, wobei die Windelhülle der ersten Inkontinenzwegwerfwindeln sich von der Windelhülle der zweiten Inkontinenzwegwerfwindeln zumindest in einer Eigenschaft, ausgewählt aus der Gruppe, Abmessungen in Längs- und/oder Querrichtung, Materialzusammensetzung, Flächengewicht des Hauptteils und oder der hinteren Seitenteile unterscheidet.

In den Figuren zeigen
Figur 1 eine Aufsicht auf eine erfindungsgemäße Inkontinenzwegwerfwindel
Figur 2 eine Schnittansicht entlang A-A der Inkontinenzwegwerfwindel nach Figur 1
Figur 3 eine Aufsicht auf eine weitere erfindungsgemäße Inkontinenzwegwerfwindel
Figur 4 eine Schnittansicht entlang A-A der Inkontinenzwegwerfwindel nach Figur 3
Figuren 5-7 Aufsichten auf weitere erfindungsgemäße Inkontinenzwegwerfwindeln mit konturierter Beinöffnung
Figuren 8 schematische Darstellungen der Bereitstellung einer Gruppe von erfindungsgemäßen ersten und zweiten Inkontinenzwegwerfwindeln
Figuren 9 schematische Darstellungen der Bereitstellung einer weiteren Gruppe von erfindungsgemäßen ersten und zweiten Inkontinenzwegwerfwindeln

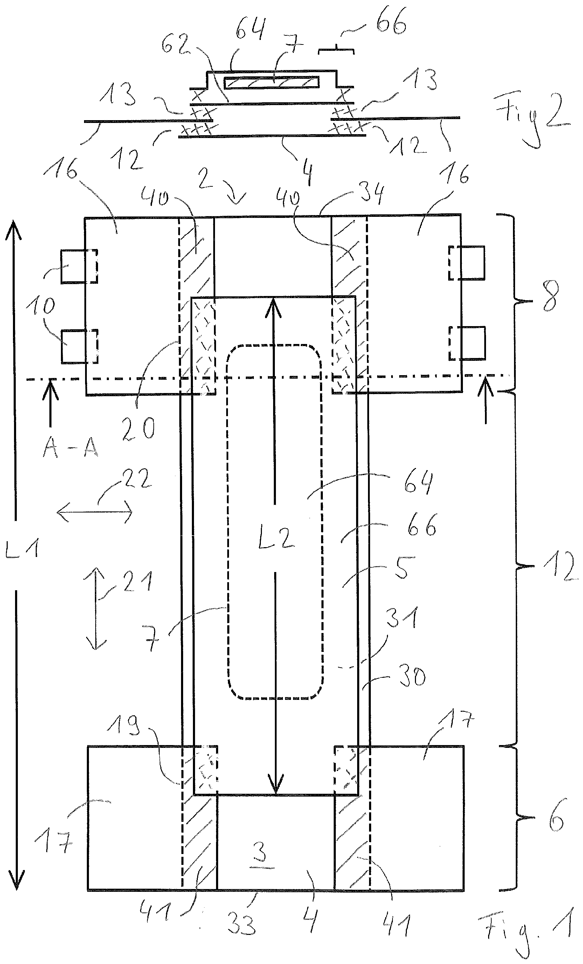

Figur 1 sowie Figur 2 (Querschnitt entlang A-A) zeigen schematisch, nicht maßstabsgerecht eine Draufsicht auf die Innenseite, also die körperzugewandte Seite einer absorbierenden Inkontinenzwegwerfwindel 2 in eben ausgefaltetem Zustand. Die Inkontinenzwegwerfwindel 2 umfasst eine äußere Windelhülle 3 mit einer in Gebrauch körperzugewandten Innenseite 30 und einer in Gebrauch körperabgewandten Außenseite 31. Die Windelhülle 3 mit der Länge L1, umfasst einen rechteckigen Hauptteil 4 mit einem Vorderbereich 6, einem Rückbereich 8 und einem in Längsrichtung 21 dazwischen liegenden Schrittbereich 12. Die Windelhülle 3 umfasst außerdem zwei hintere rechteckige Seitenteile 16 sowie zwei vordere rechteckige Seitenteile 17 und ist damit fünfteilig ausgebildet. Seitenteile 16, 17 und Hauptteil 4 sind aus Vliestoffmaterialien gebildet. Die hinteren Seitenteile 16 sind im Rückbereich 8 in überlappender Konfiguration mit ihrer Außenseite auf der Innenseite 30 des Hauptteils 4 in den ersten Überlappungsbereichen 40 (schraffiert dargestellt) festgelegt und erstrecken sich in Querrichtung 22 über hintere seitliche Längsränder 20 des Hauptteils 4 hinaus. Entsprechend sind die vorderen Seitenteile 17 im Vorderbereich 6 in zweiten Überlappungsbereichen 41 auf dem Hauptteil 4 festgelegt und erstrecken sich in Querrichtung 22 über vordere seitliche Längsränder 19 des Hauptteils 4 hinaus. Im dargestellten Fall erfolgt die Verbindung zwischen Seitenteilen 16, 17 und Hauptteil 4 in ersten und zweiten Überlappungsbereichen vermittels eines ersten Hotmeltklebstoffauftrags 12.

Die Windelhülle 3 erstreckt sich kontinuierlich über eine Länge L1 von einem vorderen Ende 33 der Windelhülle 3 über den Schrittbereich 12 bis zu einem hinteren Ende 34 der Windelhülle 3. Die Inkontinenzwegwerfwindel weist außerdem eine Absorptionseinlage 5 mit der Länge L2 auf, die auf der Innenseite der Windelhülle festgelegt ist. Die Absorptionseinlage 5 weist ein flüssigkeitsundurchlässiges Backsheet 62 und ein Topsheet 64 auf, zwischen denen ein Absorptionskörper 7 angeordnet ist. Der Absorptionskörper 7 ist zur Aufnahme und dauerhaften Speicherung von insbesondere wässrigen Körperflüssigkeiten wie Urin geeignet. Er kann in an sich üblicher Weise sogenanntes SAP (superabsorbierendes Polymer) und/oder Zellstoffasern enthalten. Der Absorptionskörper 7 kann außerdem ein- oder mehrschichtig aufgebaut sein. Das Topsheet 64 ist aus einem flüssigkeitsdurchlässigen Vliesstoffmaterial gebildet. Das Backsheet 62 ist aus einem Folienmaterial gebildet. Topsheet 64 und Backsheet 62 überfangen den Absorptionskörper 7 und erstrecken sich in Längs- und Querrichtung 21, 22 zur Bildung eines Überhangs 66 über den Absorptionskörper 7 hinaus. Der Überhang 66 der Absorptionseinlage 5 erstreckt sich in die ersten und zweiten Überlappungsbereiche 40, 41 hinein und ist dort (innerhalb der ersten bzw. zweiten Überlappungsbereiche) mit den hinteren und vorderen Seitenteilen 16, 17 zumindest abschnittsweise verbunden. Dies erfolgt derart, dass die Außenseite der jeweiligen Bereiche des Überhangs 66 auf der Innenseite der Windelhülle 3, und zwar auf der Innenseite der jeweiligen Überlappungsbereiche festgelegt wird. Im dargestellten Fall erfolgt diese Verbindung vermittels eines zweiten Hotmeltklebstoffauftrags 13 (in Figur 1 durch unterbrochene Schraffur gekennzeichnet). Die von dem Überhang 66 überfangene und dort mit dem Überhang 66 verbundene Fläche der ersten Überlappungsbereiche 40 beträgt jeweils 20 cm². Die Fläche der ersten Überlappungsbereiche beträgt jeweils 60 cm². Der Anteil der überfangenen und mit der Absorptionseinlage 5 verbundenen Fläche der ersten Überlappungsbereiche beträgt somit jeweils (das heißt in jedem der beiden Überlappungsbereiche 40) 33 1/3 %. Wie aus Figur 1 erkennbar ist der Anteil der durch den Überhang 66 überfangenen und dort mit dem Überhang 66 verbundenen Fläche der zweiten Überlappungsbereiche deutlich geringer: Die Fläche der zweiten Überlappungsbereiche 41 beträgt hier jeweils 45 cm²; die von dem Überhang 66 überfangene und dort verbundene Fläche der zweiten Überlappungsbereiche 41 beträgt jeweils 9 cm². Der Anteil der überfangenen und mit der Absorptionseinlage verbundenen Fläche der zweiten Überlappungsbereiches beträgt somit jeweils 20 %.

Die Außenseite der Absorptionseinlage kann vorzugsweise in weiteren Bereichen, also auch außerhalb der Überlappungsbereiche 40, 41, vorzugsweise auch vollflächig mit der Innenseite der Windelhülle 3 verbunden sein, insbesondere durch kontinuierlichen oder diskontinuierlichen Holtmeltklebstoffauftrag, zum Beispiel in Form eines Spiralsprühklebemusters.

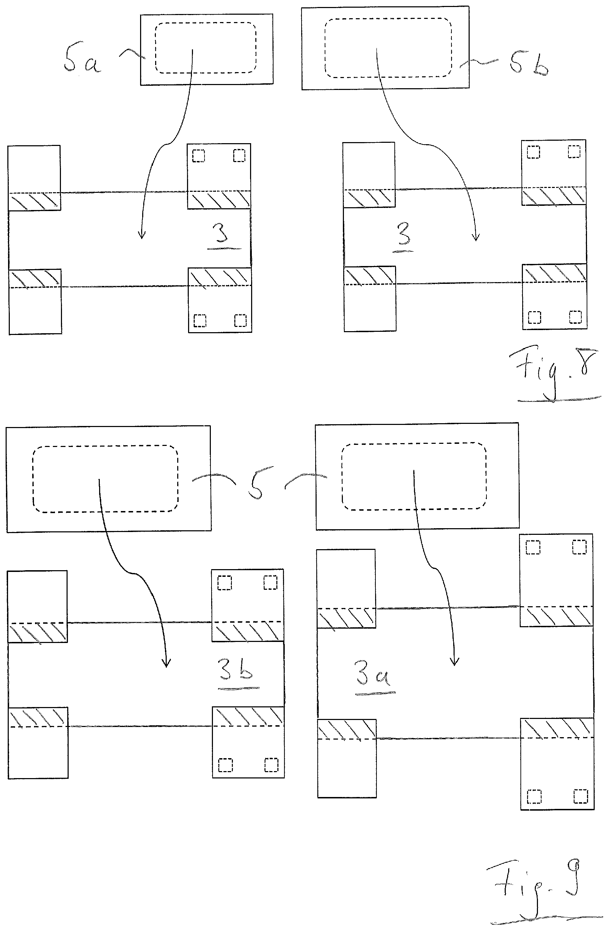

Die hinteren Seitenteile 16 weisen Verschlussmittel 10 mit Kletthaken auf, vermittels derer die hinteren Seitenteile 16 in Gebrauch auf der Außenseite der vorderen Seitenteile 17 lösbar festlegbar sind. Vorzugsweise sind die Verschlussmittel 10 außerdem auf der Außenseite des Hauptteils 4 im Vorderbereich festlegbar. Dies erlaubt es dem Nutzer, auch eine eigentlich für den Träger zu große konfektionierte Windel anzulegen, indem der Hüftumfang der Windel beim Anlegen stark reduziert wird. Die Verschlussmittel 10 können wie in Figur 1 angedeutet als Verschluss-Tapes ausgebildet sein, die sich über den äußeren Längsrand der Seitenteile hinaus erstrecken. Solchenfalls sind sie in an sich bekannter Weise vor Gebrauch und zum Zwecke der Verpackung auf sich selbst oder auf eine Innenseite der Seitenteile zurückgefaltet. Sie können alternativ als Verschluss-Pads auch vollumfänglich innerhalb der Umgangsbegrenzung der hinteren Seitenteile angeordnet sein, so wie dies schematisch in Figuren 8-9 angedeutet ist.

Unter vorderen und hinteren seitlichen Längsrändern 19, 20 des Hauptteils 4 werden im Rahmen der vorliegenden Erfindung diejenigen Längsrandbereiche des Hauptteils verstanden, an die die Seitenteile 16, 17 zur Bildung der Überlappungsbereiche angefügt sind und über welche diese sich hinaus erstrecken. Die Längserstreckung der vorderen und hinteren seitlichen Längsränder 19, 20 des Hauptteils 4 definieren damit auch die Längserstreckung des Vorderbereiches 6 und des Rückbereiches 8 der Inkontinenzwegwerfwindel 2. Im Falle, dass keine vorderen Seitenteile vorgesehen sind, wird der Vorderbereich als derjenige Abschnitt der Windelhülle definiert, welcher sich beginnend von einem vorderen Ende der Windelhülle über 30% der Länge L1 erstreckt.

Sowohl die vorderen Seitenteile 16 als auch die hinteren Seitenteile 17 als auch der Haupteil 4 sind aus einem Vliesstoffmaterial, im dargestellten Fall aus einem PP-Spinnvlies, Pegatex S, Hersteller: Pegas a.s.,Primetickä 86, 66904 Znojmo, CZ, gebildet. Das Flächengewicht des Vliesstoffmaterials der vorderen Seitenteile und des Hauptteils beträgt 30 g/m². Das Flächengewicht des Vliesstoffmaterials der hinteren Seitenteile 17 beträgt im dargestellten Fall 27 g/m².Die Faserstärke der das Vliesstoffmaterial jeweils bildenden Fasern beträgt 2 dtex.

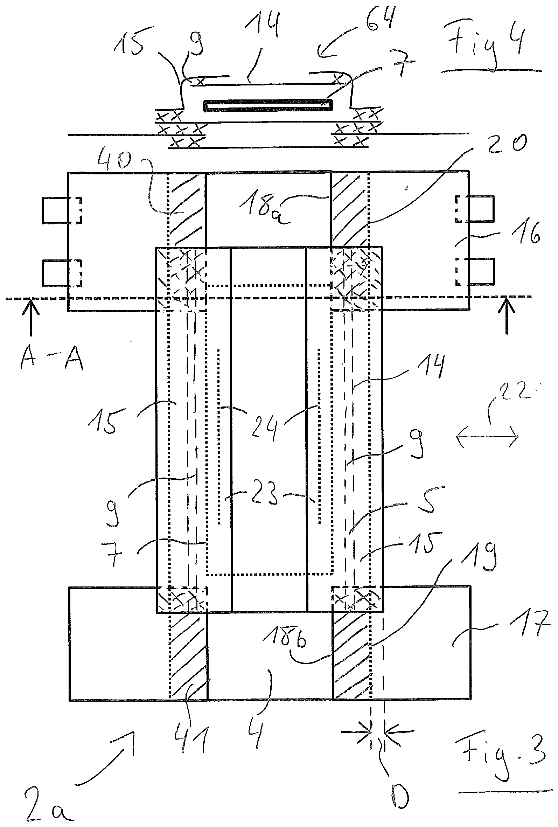

Figuren 3 und 4 zeigen eine weitere erfindungsgemäße Inkontinenzwegwerfwindel 2a. Im Folgenden werden lediglich die Komponenten und Konstruktionsmerkmale beschrieben, welche die Inkontinenzwegwerfwindel 2a von der in Figuren 1 und 2 dargestellten unterscheiden. Das Topsheet 64 der Absorptionseinlage 5 ist dreiteilig ausgebildet. Das Topsheet 64 besteht aus einem ersten mittig zentral oberhalb des Absorptionskörpers 7 angeordneten Vliesstoffmittelstreifen 14 und zwei beidseitig davon angeordneten Vliesstoffaußenstreifen 15. Die Vliesstoffstreifen 14, 15 sind vermittels drittem Hotmeltklebstoffauftrag 9, der sich vorzugsweise über die gesamte Länge der Absorptionseinlage 5 erstreckt, miteinander unlösbar verbunden. Zumindest im Schrittbereich verbleiben nach innen gerichtete distale Längsrandbereiche 23 der Vliesstoffaußenstreifen 15 jedoch unverbunden.

Diese distalen Längsrandbereiche 23 sind zumindest im Schrittbereich mit zumindest einem vorgespannten, sich in der Längsrichtung erstreckenden Elastikfaden 24 verbunden. Hierdurch können die distalen Längsrandbereiche 23 in an sich bekannter Weise von einer Oberseite (körperzugewandte Innenseite) der Absorptionseinlage 5 (in Z-Richtung) zur Bildung seitlicher Auslaufschutzbarrieren aufstehen.

Die Absorptionseinlage 5 ist in Querrichtung durchgehend breiter ausgebildet als der Hauptteil 4 der Windelhülle 3. Die Absorptionseinlage 5 überfängt daher die ersten und zweiten Überlappungsbereiche 40, 41 in Querrichtung 22 vollständig und ist dort, in den Überlappungsbereichen 40, 41 flächenhaft mit ihrer Außenseite auf der Windelhülle 3 festgelegt (unterbrochen schraffierte Bereiche in Figur 3). Folglich überlappt die Absorptionseinlage 5 entlang zumindest einer gedachten, in Querrichtung 22 verlaufenden Linie (welche im dargestellten Fall beispielhaft mit der Schnittlinie A-A zusammenfällt) sowohl einen jeweiligen inneren Längsrand 18a der hinteren Seitenteile 16 als auch einen jeweiligen hinteren seitlichen Längsrand 20 des Hauptteils 4 und ist dort auch entlang dieser Linie mit der Innenseite der Windelhülle 3 verbunden. In analoger Weise überlappt dabei die Absorptionseinlage 5 entlang zumindest einer gedachten, in Querrichtung 22 verlaufenden Linie (in der Figur 3 nicht dargestellt) sowohl einen jeweiligen inneren Längsrand 18b der vorderen Seitenteile 17 als auch einen jeweiligen vorderen seitlichen Längsrand 19 des Hauptteils 4 und ist dort auch entlang dieser Linie mit der Innenseite der Windelhülle 3 verbunden. Die die Absorptionseinlage 5 erstreckt sich dabei in Querrichtung 22 um das Maß D nach außen über die vorderen und hinteren seitlichen Längsränder 19, 20 des Hauptteils 4 hinweg. D beträgt vorliegend 15 mm.

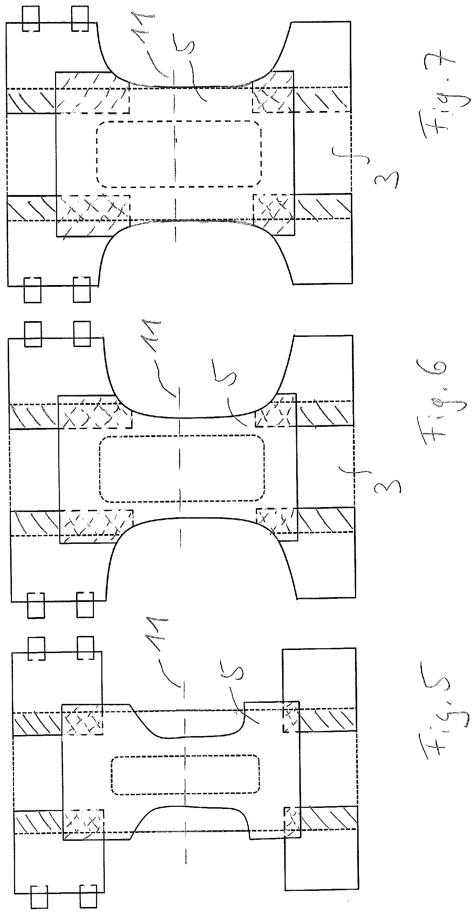

Die in Figuren 1-4 dargestellten Ausführungsformen erfindungsgemäßer Inkontinenzwegwerfwindeln weisen jeweils rechteckförmige Seitenteile 16, 17 einen rechteckförmigen Hauptteil 4 sowie eine rechteckförmige Absorptionseinlage 5 auf. Denkbar und vorteilhaft zur weiteren Verbesserung der Passform der Windel ist, die Inkontinenzwegwerfwindel mit einer konturierten, gerundeten Beinöffnungskontur zu versehen auf. Hierbei kann jeweils ein zum Schrittbereich zugewandter Querrand der hinteren und/oder vorderen Seitenteile 16, 17, die seitlichen Längsränder des Hauptteils 4, oder die Absorptionseinlage 5 mit einer zumindest abschnittsweise gerundeten Kontur versehen sein. Figuren 5-7 zeigen schematisch einige der denkbaren Ausführungsformen. So ist beispielhaft die Absorptionseinlage 5 aller Ausführungsformen nach Figuren 5-7 mit einer zumindest abschnittsweise gerundeten Beinöffnungskontur versehen, derart dass die Absorptionseinlage 5 in einem Bereich nahe einer Quermittelachse 11 der Windelhülle 3 (welche die Windelhülle in zwei gleich lange Hälften teilt) eine geringere Quererstreckung aufweist als in dem Rückbereich und dem Vorderbereich. Darüber hinaus zeigen Figuren 6 und 7 eine Inkontinenzwegwerfwindel, deren Windelhülle 3 eine zumindest abschnittsweise gerundete Beinöffnungskontur aufweist, derart dass die Windelhülle 3 in einem Bereich nahe der Quermittelachse 11 der Windelhülle 3 eine geringere Quererstreckung aufweist als in dem Rückbereich und dem Vorderbereich. Während nach Figur 7 lediglich die Seitenteile, eine gerundete Beinöffnungskontur aufweisen, zeigt Figur 6 eine sich kontinuierlich durch Seitenteile, Hauptteil und Absorptionseinlage 5 erstreckende gerundete Beinöffnungskontur.

Nach einem weiteren bevorzugten Variante der Erfindung ist vorgesehen, eine Gruppe von Inkontinenzwegwerfwindeln bereitzustellen, welche zumindest erste und zweite wie vorstehend beschriebene Inkontinenzwegwerfwindeln umfasst, wobei die ersten und zweiten Inkontinenzwegwerfwindeln hinsichtlich der Abmessungen in Längs- und Querrichtung, insbesondere auch hinsichtlich weiterer Eigenschaften wie Materialzusammensetzung oder Flächengewicht des Hauptteils und/oder der hinteren Seitenteile eine identische Windelhülle aufweisen, wobei eine erste Absorptionseinlage der ersten Inkontinenzwegwerfwindeln sich von einer zweiten Absorptionseinlage der zweiten Inkontinenzwegwerfwindeln zumindest in einer Eigenschaft, ausgewählt aus der Gruppe, Abmessungen in Längs- und/oder Querrichtung, Absorptionsvermögen (gemessen nach ISO 11948-1 (1996)), Materialzusammensetzung, Schichtaufbau, Elastifizierung unterscheidet. Es ist also vorgesehen, dass baugleiche Windelhüllen Verwendung finden für unterschiedliche Absorptionseinlagen. Figur 8 veranschaulicht schematisch die Positionierung und Fixierung erster 5a und zweiter Absorptionseinlagen 5b auf einer jeweils baugleichen, also hinsichtlich aller Eigenschaften identischen Windelhülle 3. Die erste Absorptionseinlage 5a unterscheidet sich von der zweiten Absorptionseinlage 5b zumindest hinsichtlich Ihrer Abmessungen in Längs- und Querrichtung.

Nach einer weiteren Variante ist vorgesehen, eine Gruppe von Inkontinenzwegwerfwindeln bereitzustellen, welche erste und zweite erfindungsgemäße Inkontinenzwegwerfwindeln umfasst, wobei die ersten und zweiten Inkontinenzwegwerfwindeln hinsichtlich zumindest der Abmessungen in Längs- und Querrichtung eine identische Absorptionseinlage aufweisen, wobei die Windelhülle der ersten Inkontinenzwegwerfwindeln sich von der Windelhülle der zweiten Inkontinenzwegwerfwindeln zumindest in einer Eigenschaft, ausgewählt aus der Gruppe, Abmessungen in Längs- und/oder Querrichtung, Materialzusammensetzung, Flächengewicht des Hauptteils und/oder der hinteren Seitenteile unterscheiden. Figur 9 veranschaulicht schematisch die Positionierung und Fixierung erster baugleicher Absorptionseinlagen 5 auf hinsichtlich ihrer Abmessungen in Längs- und Querrichtung sich unterscheidenden ersten 3a und zweiten Windelhüllen 3b.

## Patentansprüche

1. Absorbierende Inkontinenzwegwerfwindel (2, 2a) des offenen Typs mit einer zumindest dreiteiligen Windelhülle (3) mit einer Innenseite (30) und einer Außenseite (31), wobei die Windelhülle einen Hauptteil (4) und zumindest zwei separate hintere Seitenteile (16) umfasst, wobei der Hauptteil (4) einen Vorderbereich (6), einen Rückbereich (8) und einen in Längsrichtung (21) dazwischen liegenden zwischen den Beinen eines Benutzers zu liegen kommenden Schrittbereich (12) aufweist, und wobei die hinteren Seitenteile (16) an dem Rückbereich (8) des Hauptteils (4) beidseitig derart angeordnet sind, dass die hinteren Seitenteile (16) in ersten Überlappungsbereichen (40) mit ihrer Außenseite auf der Innenseite des Hauptteils (4) entlang hinterer seitlicher Längsränder (20) festgelegt sind, und wobei sich die hinteren Seitenteilen (16) in Querrichtung (22) über die hinteren seitlichen Längsränder (20) des Hauptteils (4) hinaus erstrecken, und wobei die hinteren Seitenteile (16) Verschlussmittel (10) aufweisen, und wobei der Hauptteil (4) und die hinteren Seitenteile (16)ein Vliesstoffmaterial umfassen oder daraus gebildet sind, und wobei die Windelhülle (3) sich kontinuierlich über eine Länge L1 von einem vorderen Ende (33) der Windelhülle (3) über den Schrittbereich (12) bis zu einem hinteren Ende (34) der Windelhülle (3) erstreckt, und wobei auf der Innenseite (30) der Windelhülle (3) eine Absorptionseinlage (5) mit einer Innenseite und einer Außenseite angeordnet ist, wobei die Absorptionseinlage (5) ein flüssigkeitsundurchlässiges Backsheet (62) und ein Topsheet (64) umfasst, zwischen denen ein Absorptionskörper (7) angeordnet ist, wobei die Absorptionseinlage (5) sich in die ersten Überlappungsbereiche (40) hinein erstreckt und dort mit ihrer Außenseite auf der Windelhülle (3) zumindest abschnittsweise festgelegt ist, wobei die Absorptionseinlage (5) eine Länge L2 aufweist und wobei gilt L2 < L1.

2. Absorbierende Inkontinenzwegwerfwindel (2, 2a) nach Anspruch 1 mit an den Vorderbereich (6) des Hauptteils (4) angeordneten, voneinander separaten vorderen Seitenteilen (17), wobei die vorderen Seitenteile ((17) an dem Vorderbereich (6) des Hauptteils (4) beidseitig derart angeordnet sind, dass die vorderen Seitenteile (17) in zweiten Überlappungsbereichen (41) mit ihrer Außenseite auf der Innenseite des Hauptteils (4) entlang vorderer seitlicher Längsränder (19) festgelegt sind, und wobei sich die vorderen Seitenteile (17) in Querrichtung (22) über die vorderen seitlichen Längsränder (19) des Hauptteils (4) hinaus erstrecken.

3. Absorbierende Inkontinenzwegwerfwindel (2, 2a) nach Anspruch 2, wobei die Absorptionseinlage (5) sich in die zweiten Überlappungsbereiche (41) hinein erstreckt und dort mit ihrer Außenseite auf der Windelhülle (3) zumindest abschnittsweise festgelegt ist.

4. Absorbierende Inkontinenzwegwerfwindel (2, 2a) nach Anspruch 1, 2 oder 3, wobei das Backsheet (62) und/oder das Topsheet (64) in Querrichtung (22) und/oder in Längsrichtung (15) einen Überhang (66) über den Absorptionskörper (7) bilden und Teilbereiche des Überhangs (66) sich in die ersten und/oder zweiten Überlappungsbereiche (40, 41) hinein erstrecken und dort mit ihrer Außenseite auf der Windelhülle (3) zumindest abschnittsweise festgelegt ist..

5. Absorbierende Inkontinenzwegwerfwindel (2, 2a) nach einem der vorgenannten Ansprüche, **dadurch gekennzeichnet, dass** mindestens 20%, insbesondere mindestens 30%, weiter insbesondere höchstens 90 %, weiter insbesondere höchstens 80% der Fläche der ersten Überlappungsbereiche (40) mit der Absorptionseinlage (5) verbunden ist.

6. Absorbierende Inkontinenzwegwerfwindel (2, 2a) nach einem der vorgenannten Ansprüche, wobei das Verhältnis L2/L1 < 0,9, insbesondere < 0,8, weiter insbesondere < 0,7, weiter insbesondere > 0,3 ist.

7. Absorbierende Inkontinenzwegwerfwindel (2, 2a) nach einem der vorgenannten Ansprüche 2-5, **dadurch gekennzeichnet, dass** mindestens 10%, insbesondere mindestens 20%, weiter insbesondere höchstens 85 %, weiter insbesondere höchstens 75% der Fläche der zweiten Überlappungsbereiche (41) mit der Absorptionseinlage (5) verbunden ist.

8. Absorbierende Inkontinenzwegwerfwindel (2, 2a) nach einem der vorgenannten Ansprüche 2-6, **dadurch gekennzeichnet, dass** der Anteil der mit der Absorptionseinlage (5) verbunden Fläche der zweiten Überlappungsbereiche (41) jeweils geringer ist als der Anteil der mit der Absorptionseinlage (5) verbundenen Fläche der ersten Überlappungsbereiche (40).

9. Absorbierende Inkontinenzwegwerfwindel (2, 2a) nach einem der vorgenannten Ansprüche, **dadurch gekennzeichnet, dass** sich der Absorptionskörper (7) nicht in die ersten und/oder zweiten Überlappungsbereiche (40, 41) hinein erstreckt.

10. Absorbierende Inkontinenzwegwerfwindel (2, 2a) nach einem der vorgenannten Ansprüche, **dadurch gekennzeichnet, dass** die Absorptionseinlage(5) die ersten und/oder zweiten Überlappungsbereiche (40, 41) in Querrichtung (22) vollständig überfängt.

11. Absorbierende Inkontinenzwegwerfwindel (2, 2a) nach einem der vorgenannten Ansprüche, **dadurch gekennzeichnet, dass** die Absorptionseinlage 5 eine zumindest abschnittsweise gerundete, also kurvenförmige Beinöffnungskontur aufweist, derart; dass die Absorptionseinlage (5) in einem Bereich auf Höhe einer Quermittelachse (11) der Windelhülle (3) eine geringere Quererstreckung aufweist als in dem Rückbereich (8) und/oder Vorderbereich (6).

12. Absorbierende Inkontinenzwegwerfwindel (2, 2a) nach einem der vorgenannten Ansprüche, **dadurch gekennzeichnet, dass** die Windelhülle (3) eine zumindest abschnittsweise gerundete, also kurvenförmige Beinöffnungskontur aufweist, derart dass die Windelhülle (3) in einem Bereich auf Höhe der Quermittelachse (11) der Windelhülle (3) eine geringere Quererstreckung aufweist als in dem Rückbereich (8) und/oder Vorderbereich (6).

13. Gruppe von Inkontinenzwegwerfwindeln, welche zumindest erste und zweite wie Inkontinenzwegwerfwindeln nach einem der vorstehenden Ansprüche umfasst, wobei die ersten und zweiten Inkontinenzwegwerfwindeln hinsichtlich der Abmessungen in Längs- und/oder Querrichtung, insbesondere auch hinsichtlich weiterer Eigenschaften wie Materialzusammensetzung oder Flächengewicht des Hauptteils und/oder der hinteren Seitenteile, insbesondere hinsichtlich aller Eigenschaften eine identische Windelhülle (3) aufweisen, wobei die Absorptionseinlage (5a) der ersten Inkontinenzwegwerfwindeln sich von der Absorptionseinlage (5b) der zweiten Inkontinenzwegwerfwindeln zumindest in einer Eigenschaft, ausgewählt aus der Gruppe, Abmessungen in Längs- und/oder Querrichtung, Absorptionsvermögen (gemessen nach ISO 11948-1 (1996)) Materialzusammensetzung, Schichtaufbau, Elastifizierung unterscheidet.

14. Gruppe von Inkontinenzwegwerfwindeln, welche zumindest erste und zweite Inkontinenzwegwerfwindeln nach einem der vorstehenden Ansprüche umfasst, wobei die ersten und zweiten Inkontinenzwegwerfwindeln hinsichtlich der Abmessungen in Längs- und/oder Querrichtung, und vorzugsweise auch hinsichtlich weiterer Eigenschaften wie Materialzusammensetzung oder Flächengewicht, weiter insbesondere hinsichtlich aller Eigenschaften eine identische Absorptionseinlage (5) aufweisen, wobei die Windelhülle (3a) der ersten Inkontinenzwegwerfwindeln sich von der Windelhülle (3b) der zweiten Inkontinenzwegwerfwindeln zumindest in einer Eigenschaft, ausgewählt aus der Gruppe, Abmessungen in Längs- und/oder Querrichtung, Materialzusammensetzung, Flächengewicht des Hauptteils und/oder der hinteren Seitenteile unterscheiden.

## Claims

1. Absorbent disposable incontinence diaper (2, 2a) of the open type, having an at least tripartite diaper shell (3) which has an inner side (30) and an outer side (31), wherein the diaper shell comprises a main part (4) and at least two separate rear lateral parts (16), wherein the main part (4) has a front region (6), a rear region (8), and a crotch region (12) which in the longitudinal direction (21) is therebetween so as to come to lie between the legs of a user, and wherein the rear lateral parts (16) are disposed on both sides of the rear region (8) of the main part (4) in such a manner that the rear lateral parts (16), by way of their outer side, are fixed in first overlapping regions (40) on the inner side of the main part (4) along rear lateral longitudinal peripheries (20), and wherein the rear lateral parts (16) in the transverse direction (22) extend beyond the rear lateral longitudinal peripheries (20) of the main part (4), and wherein the rear lateral parts (16) have closure means (10), and wherein the main part (4) and the rear lateral parts (16) comprise a nonwoven material or are formed therefrom, and wherein the diaper shell (3) continuously extends across a length L1, from a front end (33) of the diaper shell (3) across the crotch region (12) to a rear end (34) of the diaper shell (3), and wherein an absorbent insert (5) having an inner side and an outer side is disposed on the inner side (30) of the diaper shell (3), wherein the absorbent insert (5) comprises a fluid-impermeable back sheet (62) and a top sheet (64) between which an absorbent body (7) is disposed, wherein the absorbent insert (5) extends into the first overlapping regions (40) and there, by way of its outer side, is at least in portions fixed on the diaper shell (3), wherein the absorbent insert (5) has a length L2, and wherein L2 < L1 applies.

2. Absorbent disposable incontinence diaper (2, 2a) according to Claim 1, having front lateral parts (17) which are separate from one another and disposed on the front region (6) of the main part (4), wherein the front lateral parts (17) are disposed on both side of the front region (6) of the main part (4) in such a manner that the front lateral parts (17), by way of their outer side, are fixed in second overlapping regions (41) on the inner side of the main part (4) along front lateral longitudinal peripheries (19), and wherein the front lateral parts (17) in the transverse direction (22) extend beyond the front lateral longitudinal peripheries (19) of the main part (4).

3. Absorbent disposable incontinence diaper (2, 2a) according to Claim 2, wherein the absorbent insert (5) extends into the second overlapping regions (41) and there, by way of its outer side, is at least in portions fixed on the diaper shell (3).

4. Absorbent disposable incontinence diaper (2, 2a) according to Claim 1, 2, or 3, wherein the back sheet (62) and/or the top sheet (64) in the transverse direction (22) and/or in the longitudinal direction (15) form an overhang (66) over the absorbent body (7), and part-regions of the overhang (66) extend into the first and/or second overlapping regions (40, 41) and there, by way of their outer side, are at least in portions fixed on the diaper shell (3).

5. Absorbent disposable incontinence diaper (2, 2a) according to one of the preceding claims, **characterized in that** at least 20%, particularly at least 30%, furthermore particularly at most 90%, furthermore particularly at most 80% of the area of the first overlapping regions (40) is connected to the absorbent insert (5).

6. Absorbent disposable incontinence diaper (2, 2a) according to one of the preceding claims, wherein the ratio L2/L1 is < 0.9, particularly < 0.8, furthermore particularly < 0.7, furthermore particularly > 0.3.

7. Absorbent disposable incontinence diaper (2, 2a) according to one of preceding Claims 2 to 5, **characterized in that** at least 10%, particularly at least 20%, furthermore particularly at most 85%, furthermore particularly at most 75% of the area of the second overlapping regions (41) is connected to the absorbent insert (5).

8. Absorbent disposable incontinence diaper (2, 2a) according to one of preceding Claims 2 to 6, **characterized in that** the proportion of that area of the second overlapping regions (41) that is connected to the absorbent insert (5) is in each case smaller than the proportion of that area of the first overlapping regions (40) that is connected to the absorbent insert (5).

9. Absorbent disposable incontinence diaper (2, 2a) according to one of the preceding claims, **characterized in that** the absorbent body (7) does not extend into the first and/or second overlapping regions (40, 41).

10. Absorbent disposable incontinence diaper (2, 2a) according to one of the preceding claims, **characterized in that** the absorbent insert (5) completely overlies the first and/or second overlapping regions (40, 41) in the transverse direction (22).

11. Absorbent disposable incontinence diaper (2, 2a) according to one of the preceding claims, **characterized in that** the absorbent insert (5) has a leg-opening contour which is, at least in portions, rounded, that is to say curved, in such a manner that the absorbent insert (5) in a region which is level with a transverse center axis (11) of the diaper shell (3) has a smaller transverse extent than in the rear region (8) and/or the front region (6).

12. Absorbent disposable incontinence diaper (2, 2a) according to one of the preceding claims, **characterized in that** the diaper shell (3) has a leg-opening contour which is, at least in portions, rounded, that is to say curved, in such a manner that the diaper shell (3) in a region which is level with the transverse center axis (11) of the diaper shell (3) has a smaller transverse extent than in the rear region (8) and/or the front region (6).

13. Group of disposable incontinence diapers, which comprises at least first and second such disposable incontinence diapers according to one of the preceding claims, wherein the first and second disposable incontinence diapers in respect of the dimensions in the longitudinal and/or transverse directions, in particular also in respect of further properties such as material composition or area weight of the main part and/or the rear lateral parts, in particular in respect of all properties have an identical diaper shell (3), wherein the absorbent insert (5a) of the first disposable incontinence diapers differs from the absorbent insert (5b) of the second disposable incontinence diapers at least in one property selected from the group of dimensions in the longitudinal and/or transverse directions, absorptivity (measured according to ISO 11948-1 (1996)), material composition, layer construction, elastification.

14. Group of disposable incontinence diapers, which comprises at least first and second disposable incontinence diapers according to one of the preceding claims, wherein the first and second disposable incontinence diapers have an absorbent insert (5) which is identical in respect of the dimensions in the longitudinal and/or transverse directions, and preferably also in respect of further properties such as material composition or area weight, furthermore particularly in respect of all properties, wherein the diaper shell (3a) of the first disposable incontinence diapers differs from the diaper shell (3b) of the second disposable incontinence diapers at least in one property selected from the group of dimensions in the longitudinal and/or transverse directions, material composition, area weight of the main part and/or of the rear lateral parts.

## Revendications

1. Couche incontinence absorbante jetable (2, 2a) du type ouvert, comportant une enveloppe de couche (3) en au moins trois pièces, comportant une face intérieure (30) et une face extérieure (31), l'enveloppe de couche comprenant une pièce principale (4) et au moins deux pièces latérales arrière distinctes (16), la pièce principale (4) présentant une zone avant (6), une zone arrière (8) et une zone d'enfourchure (12), disposée entre elles dans la direction longitudinale (21), et venant se trouver entre les jambes d'un utilisateur, et les pièces latérales arrière (16) étant disposées des deux côtés contre la zone arrière (8) de la pièce principale (4) de telle sorte que les pièces latérales arrière (16) soient, dans les premières zones de chevauchement (40), fixées par leur face arrière sur la face intérieure de la pièce principale (4) le long des bords longitudinaux latéraux arrière (20), et les pièces latérales arrière (16) s'étendant dans la direction transversale (22) au-delà des bords longitudinaux latéraux arrière (20) de la pièce principale (4), et les pièces latérales arrière (16) comprenant des attaches (10), et la pièce principale (4) et les pièces latérales arrière (16) comprenant un matériau non-tissé ou en étant constituées, et l'enveloppe de couche (3) s'étendant d'une manière continue sur une longueur L1, d'une extrémité avant (33) de l'enveloppe de couche (3) à une extrémité arrière (34) de l'enveloppe de couche (3) en passant par la zone d'enfourchure (12), et un matelas absorbant (5), ayant une face intérieure et une face extérieure, étant disposé sur la face intérieure (30) de l'enveloppe de couche (3), le matelas absorbant (5) comprenant un feuillet arrière (62) et un feuillet supérieur (64) imperméables aux liquides, entre lesquels est disposé un corps absorbant (7), le matelas absorbant (5) s'étendant à l'intérieur des premières zones de chevauchement (40), et y étant fixé, au moins par segments, par sa face arrière sur l'enveloppe de couche (3), le matelas absorbant (5) ayant une longueur L2, avec L2 < L1.

2. Couche incontinence absorbante jetable (2, 2a) selon la revendication 1, comportant des pièces latérales avant (17), distinctes l'une de l'autre, disposées contre la zone avant (6) de la pièce principale (4), les pièces latérales avant (17) étant disposées des deux côtés contre la zone avant (6) de la pièce principale (4) de telle sorte que les pièces latérales avant (17) soient, dans des deuxièmes zones de chevauchement (41), fixées par leur face extérieure sur la face intérieure de la pièce principale (4) le long de bords longitudinaux latéraux avant (19), et les pièces latérales avant (17) s'étendant dans la direction transversale (22) au-delà des bords longitudinaux latéraux avant (19) de la pièce principale (4).

3. Couche incontinence absorbante jetable (2, 2a) selon la revendication 2, dans laquelle le matelas absorbant (5) s'étend dans l'intérieur des deuxièmes zones de chevauchement (41) et y est fixé, au moins par segments, par sa face extérieure sur l'enveloppe de couche (3).

4. Couche incontinence absorbante jetable (2, 2a) selon la revendication 1, 2 ou 3, dans laquelle le feuillet arrière (62) et/ou le feuillet supérieur (64) forment dans la direction transversale (22) et/ou dans la direction longitudinale (15) un surplomb (66) au-delà du corps absorbant (7), et des zones partielles du surplomb (66) s'étendent à l'intérieur de la première et/ou la deuxième zones de chevauchement (40, 41) et y sont au moins par segments fixées par leur face extérieure sur l'enveloppe de couche (3).

5. Couche incontinence absorbante jetable (2, 2a) selon l'une des revendications précédentes, **caractérisée en ce qu'**au moins 20 %, en particulier au moins 30 %, plus particulièrement au plus 90 %, plus particulièrement au plus 80 % de l'aire des premières zones de chevauchement (40) sont assemblées au matelas absorbant (5).

6. Couche incontinence absorbante jetable (2, 2a) selon l'une des revendications précédentes, dans laquelle le rapport L2/L1 est < 0,9, en particulier < 0,8, plus particulièrement < 0,7, plus particulièrement > 0,3.

7. Couche incontinence absorbante jetable (2, 2a) selon l'une des revendications précédentes 2 à 5, **caractérisée en ce qu'**au moins 10 %, en particulier au moins 20 %, plus particulièrement au plus 85 %, plus particulièrement au plus 75 % de l'aire des deuxièmes zones de chevauchement (41) sont assemblées au matelas absorbant (5).

8. Couche incontinence absorbante jetable (2, 2a) selon l'une des revendications précédentes 2 à 6, **caractérisée en ce que** la proportion de l'aire de chacune des deuxièmes zones de chevauchement (41) assemblée au matelas absorbant (5) est plus petite que la proportion de l'aire des premières zones de chevauchement (40) assemblée au matelas absorbant (5).

9. Couche incontinence absorbante jetable (2, 2a) selon l'une des revendications précédentes, **caractérisée en ce que** le corps absorbant (7) ne s'étend pas à l'intérieur les premières et/ou les deuxièmes zones de chevauchement (40, 41).

10. Couche incontinence absorbante jetable (2, 2a) selon l'une des revendications précédentes, **caractérisée en ce que** le matelas absorbant (5) recouvre complètement dans la direction transversale (22) les premières et/ou les deuxièmes zones de chevauchement (40, 41).

11. Couche incontinence absorbante jetable (2, 2a) selon l'une des revendications précédentes, **caractérisée en ce que** le matelas absorbant (5) comprend un contour d'ouverture des jambes qui au moins par segments est arrondi, c'est-à-dire de forme incurvée, de telle sorte que le matelas absorbant (5) présente dans une zone à la hauteur d'un axe central transversale (11) de l'enveloppe de couche (3) une étendue transversale plus petite que dans la zone arrière (8) et/ou dans la zone avant (6).

12. Couche incontinence absorbante jetable (2, 2a) selon l'une des revendications précédentes, **caractérisée en ce que** l'enveloppe de couche (3) comprend un contour d'ouverture des jambes qui au moins par segments est arrondi, c'est-à-dire de forme incurvée, de telle sorte que l'enveloppe de couche (3) présente dans une zone à la hauteur de l'axe central longitudinal (11) de l'enveloppe de couche (3) une étendue transversale plus petite que dans la zone arrière (8) et/ou la zone avant (6).

13. Groupe de couches incontinence absorbantes jetables, qui comprend au moins une première et une deuxième couches incontinence absorbantes jetables selon l'une des revendications précédentes, la première et la deuxième couches incontinence jetables présentant, pour ce qui est des dimensions dans la direction longitudinale et/ou dans la direction transversale, en particulier aussi pour ce qui est d'autres propriétés telles que la composition en matériaux ou l'aire surfacique de la pièce principale et/ou des pièces latérales arrière, en particulier pour ce qui est de toutes les propriétés, une enveloppe de couche identique (3), le matelas absorbant (5a) des premières couches incontinence absorbantes jetables se distinguant du matelas absorbant (5b) des deuxièmes couches incontinence absorbantes jetables au moins par une propriété choisie dans le groupe consistant en les dimensions dans la direction longitudinale et/ou la direction transversale, le pouvoir absorbant (mesuré selon ISO 11948-1 (1996)), la composition en matériaux, la structure de couches, l'élastification.

14. Groupe de couches incontinence absorbantes jetables, qui comprend au moins des premières et des deuxièmes couches incontinence absorbantes jetables selon l'une des revendications précédentes, dans lequel les premières et les deuxièmes couches incontinence absorbantes jetables présentent, pour ce qui est des dimensions dans la direction longitudinale et/ou dans la direction transversale, et de préférence aussi pour ce qui est d'autres propriétés telles que la composition en matériaux ou l'aire surfacique, plus particulièrement pour ce qui est de toutes les propriétés, un matelas absorbant identique (5), l'enveloppe de couche (3a) des premières couches incontinence à jeter se distinguant de l'enveloppe de couche (3b) des deuxièmes couches incontinence jetables au moins par une propriété choisie dans le groupe consistant en les dimensions dans la direction longitudinale et/ou la direction transversale, la composition en matériaux, l'aire surfacique de la pièce principale et/ou des pièces latérales arrière.
